**Europäisches Patentamt**

**European Patent Office**

**Office européen des brevets**

(19)

(11) Veröffentlichungsnummer: **0 231 777 B1**

(12) **EUROPÄISCHE PATENTSCHRIFT**

(45) Veröffentlichungstag der Patentschrift:
27.03.91 Patentblatt 91/13

(51) Int. Cl.⁵: **A61K 7/00, A61K 7/48,**
A61K 7/075, A61K 31/355,
C07D 311/72

(21) Anmeldenummer: 87100429.7

(22) Anmeldetag: 15.01.87

(54) Feuchthaltende kosmetische Mittel und deren Verwendung.

(30) Priorität: 28.01.86 US 823214

(43) Veröffentlichungstag der Anmeldung:
12.08.87 Patentblatt 87/33

(45) Bekanntmachung des Hinweises auf die
Patenterteilung:
27.03.91 Patentblatt 91/13

(84) Benannte Vertragsstaaten:
CH DE FR GB LI

(56) Entgegenhaltungen:
EP-A- 0 158 090
EP-A- 0 165 457
DE-A- 2 144 249
DE-C- 866 830
US-A- 4 000 276
US-A- 4 525 344

(56) Entgegenhaltungen:
CHEMICAL ABSTRACTS, Vol. 99, Nr. 1, 4. Juli
1983, Columbus, Ohio, USA; MIDORI KAGAKU
KENKYUSHO Y.K. "Vitramin E linolate" Seite
539, Zusammenfassung-Nr. 5879x
PATENT ABSTRACTS OF JAPAN, unexamined
applications, Sektion C, Vol. 2, Nr. 59, 27. April
1978 THE PATENT OFFICE JAPANESE GO-
VERNMENT Seite 393 C 78
AUSTRIA-CODEX 1984/1985, Oesterreichischer Apotheker-Verlag, Wien 1984 Seite 632

(73) Patentinhaber: F. HOFFMANN-LA ROCHE AG
Postfach 3255
CH-4002 Basel (CH)

(72) Erfinder: Djerassi, David
205 Third Avenue
New York, N.Y. 10003 (US)
Erfinder: Schnurman, Errol Sheldon
65 Fleetwood Drive
Rockaway, N.J. 07866 (US)

(74) Vertreter: Urech, Peter, Dr. et al
Grenzacherstrasse 124 Postfach 3255
CH-4002 Basel (CH)

Festiger, Deodorantien, Haarpflegemittel, Puder und Seifen.

Zusätzlich zu den oben genannten kosmetisch wirksamen Bestandteilen können die kosmetischen Mittel mit feuchthaltenden Eigenschaften auch noch herkömmliche Exzipentien und Zusatzstoffe enthalten, die zur Herstellung von topisch applizierbaren kosmetischen Mitteln (häufig) verwendet werden, z.B. Konservierungsmittel, Verdickungsmittel, Emulgatoren, Viskositätsstabilisatoren, Geschmacksstoffe, Farbstoffe und Parfüms. Zudem können herkömmliche Antioxidantien, wie beispielsweise butyliertes Hydroxyanisol (BHA), butyliertes Hydroxytoluol (BHT) und 6-Aethoxy-1,2-dihydro-2,2,4-trimethylchinolin (Ethoxyquin), in diesen Mitteln vorhanden sein.

Das kosmetische Mittel kann herkömmliche kosmetisch verträgliche Trägerstoffe zur topischen Applikation enthalten.

In denjenigen erfindungsgemässen kosmetischen Mitteln, die weitere kosmetisch wirksame Bestandteile aufweisen, beträgt die Menge feuchthaltenden Stoffes im allgemeinen von 0,1 bis 15 Gewichtsprozent der Gesamtmenge des Mittels, wobei die eigentliche Menge des feuchthaltenden Bestandteils vom gewünschten Grad der Förderung der Feuchthaltung abhängt. Im Falle der Benützung von niedrigeren Mengen als 0,1 Gewichtsprozent wird eine geringe feuchthaltende Wirkung erzielt. Grössere Mengen als 15 Gewichtsprozent des feuchthaltenden Stoffes können benützt werden, jedoch werden im allgemeinen so keine besseren feuchthaltenden Ergebnisse erzielt als durch Mittel, die 15 Gewichtsprozent des feuchthaltenden Stoffes enthalten. Vorzugsweise enthalten solche kosmetische Mittel 1 Gewichtsprozent bis 5 Gewichtsprozent feuchthaltenden Stoffes. Zum Erreichen der erwünschten kosmetischen Wirkung auf der Haut sind die kosmetischen Wirkstoffe zweckmässigerweise in üblichen Mengen in den Mitteln vorhanden.

In denjenigen erfindungsgemässen kosmetischen Mitteln, die nur zur Feuchthaltung der Haut verwendet werden, d.h. die keine zusätzlichen kosmetischen Wirkstoffe enthalten, beträgt die Menge des oben definierten feuchthaltenden Stoffes vorzugsweise 30 Gewichtsprozent bis 75 Gewichtsprozent, wobei der Rest des Mittels aus kosmetisch verträglichem organischem Trägerstoff, insbesondere kosmetisch verträglichem organischem Lösungsmittel, besteht. Die feuchthaltenden Bestandteile befinden sich normalerweise im Lösungsmittel als Lösung oder Emulsion. Herkömmliche kosmetisch verträgliche organische Lösungsmittel können zu diesem Zwecke benützt werden. Zu den bevorzugten organischen Lösungsmitteln gehören Oele, wie Mineralöle, Fettsäure-niederalkylester, insbesondere die $C_{1-7}$-Alkylester, z.B. Methyl-, Aethyl-, n-Propyl- und Isopropylester, sowie Alkohole, insbesondere niedere Alkanole, wie Methyl-, Aethyl- oder Isopropylalkohol. Diese Mittel können zudem herkömmliche Exzipentien enthalten, wie Stabilisatoren, Parfüms, Emulgatoren und Konservierungsmittel. Ansonsten kann auch bloss organisches Lösungsmittel vorhanden sein.

Die erfindungsgemässen Mittel halten die Haut aber nicht nur feucht, sondern verleihen der Haut zudem Festigkeit, Elastizität, Glätte und Zartheit. Nach wiederholter Applikation der erfindungsgemässen Mittel erscheint der Zustand des Hautgewebes sichtlich verbessert ("retexturized").

Gemäss der bevorzugten Ausführungsform dieser Erfindung erzielt man durch Verwendung des unten definierten Gemisches von Fettsäureestern eine ganz neuartige und unerwartete Feuchthaltung.

Die angegebenen Gewichtsprozente beziehen sich auf das Gesamtgewicht der im feuchthaltenden Stoff vorhandenen α-Tocopherol-fettsäureester :

| α-Tocopherol | Gewichtsprozent | | |
|---|---|---|---|
| Linolsäureester | 30% | bis | 70% |
| Oelsäureester | 15% | bis | 40% |
| Linolensäureester | 2% | bis | 30% |
| Palmitinsäureester | 0,5% | bis | 20% |
| Myristinsäureester | 0,1% | bis | 10% |
| Stearinsäureester | 0,1% | bis | 10% |

Bevorzugt sind in diesen Gemischen 40 bis 60 Gewichtsprozent α-Tocopherol-linolsäureester und 20 bis 30 Gewichtsprozent α-Tocopherol-ölsäureester vorhanden. Im allgemeinen verursachen Gemische der α-Tocopherol-fettsäureester, falls diese topisch appliziert werden, wenig oder gar keine Reizung der menschlichen Haut.

Die Erfindung wird durch die nachfolgenden Beispiele veranschaulicht :

3

## I. Herstellung der feuchthaltenden Bestandteile

### Beispiel 1

Im folgenden ist unter dem Ausdruck "Gemisch von Fettsäuren" ein Gemisch der nachfolgenden Zusammensetzung zu verstehen :

| Fettsäure | Gewichtsprozent der Fettsäure |
|---|---|
| Linolsäure | 65,5% |
| Oelsäure | 19,5% |
| Linolensäure | 10,5% |
| Palmitinsäure | 3,5% |
| Myristinsäure | 0,5% |
| Stearinsäure | 0,5% |

in einem mit einem Thermometer, einem Tropftrichter, einem Kühler, einem mechanischen Rührer sowie einem Absorptionsgefäss versehenen 5L-Dreihalsrundkolben wurden 646 g (2,29 Mol) des Gemisches von Fettsäuren und 2,0 L Toluol vorgelegt. Die hellgelbe Lösung wurde auf einem Dampfbad auf 45°C erwärmt. während 400 g (3,36 Mol) Thionylchlorid innerhalb von 15 Minuten zugegeben wurden. Die Lösung wurde weitere 3 Stunden bei 90°C gerührt. Dann wurde da überschüssige Thionylchlorid durch Destillieren bei 26-28 mm Hg (3466-3733 Pa) und 50°C entfernt. Das zurückbleibende dunkle Oel wurde zweimal mit jeweils 500 ml bis 1,5 L n-Hexan verrieben. Man engte das Gemisch nach jedem Verreiben zu einem Oel ein, was 687 g (2,29 Mol) der Säurechloride des obigen Gemisches von Fettsäuren ergab.

Anschliessend wurden im einem zweiten 5L-Dreihalsrundkolben 984 g (2,28 Mol) α-Tocopherol, 1,4 L Methylenchlorid und 734 ml Pyridin vorgelegt. Man rührte die resultierende Lösung bei Raumtemperatur und gab innerhalb von 20 Minuten eine Lösung der 687 g (2,29 Mol) oben angegebenen Säurechloride in 1,0 L Methylenchlorid zu. Bis auf 54°C war die Reaktion exotherm. Das Gemisch wurde 3 Stunden gerührt und anschliessend die Reaktion durch Zugabe in ein gerührtes Gemisch von 2,0 L Wasser und 2,0 L Methylenchlorid zum Stillstand gebracht. Man wusch die organische Schicht zweimal mit jeweils 1,0 bis 2,0 L 3N Salzsäure, gefolgt von 2,0 L gesättigter Natriumchloridlösung. Die organische Schicht wurde bei 26-28 mm Hg (3466-3733 Pa) und 50°C in einem Rotationsverdampfer eingeengt, was 1,58 kg rohes Gemisch von α-Tocopherol-fettsäureestern (rohes Estergemisch) in Form eines dunklen zähflüssigen Oels ergab. Das rohe Estergemisch wurde unter Verwendung von 3,4 kg kieselgel (400 Mesh), das auf einem grossen Glasfritte aufgetragen war, gereinigt. Diese wiederverwendete Schicht kieselgel wurde mit 1,58 kg des rohen Estergemisches, gelöst in 1,5 L Diäthyläther/n-Hexan (1 : 9), imprägniert. Dann eluierte man die Kieselgelschicht mit 15,0 L Diäthyläther/n-Hexan (1 : 9) unter vermindertem Druck (70 mm Hg, 9333 Pa) in fünf Kolben.

| | Menge | Qualität |
|---|---|---|
| Fraktion 1 | 1,0 L | Reines Gemisch von Estern |
| Fraktion 2 | 3,5 L | Reines Gemisch von Estern |
| Fraktion 3 | 3,5 L | Estergemisch sowie Amidsäure |
| Fraktion 4 | 3,5 L | Rohe Linolsäure |
| Fraktion 5 | 3,5 L | Rohe Linolsäure |

Fraktionen 1 und 2 wurden vereinigt und bei 26-28 mm Hg (3466-3733 Pa) und 55°C eingeengt, was 1,0 kg gemischte α-Tocopherol-fettsäureester als bernsteinfarbenes Oel ergab. Fraktion 3 wurde bei 26-28 mm Hg (3466-3733 Pa) und 55°C zu einem dunklen Oel eingeengt, das aus einem rohen Estergemisch bestand. Dieses Estergemisch (395 g) wurde in 500 ml n-Hexan gelöst und die Lösung auf eine neue Schicht kieselgel 60 (500 g, 230-400 Mesh) vorgelegt, und die Säule wurde mit 2,0 L n-Hexan eluiert. Das Eluat wurde bei 26-28 mm Hg (3466-3733 Pa) und 55°C eingeengt, was 310 g eines Gemisches von α-Tocopherol-fettsäureestern als bernsteinfarbenes Oel ergab.

Sämtliche Fraktionen wurden vereinigt und innerhalb von 3 Stunden bei 0,5 mm Hg (66,6 Pa) und 70°C eingeengt, was 1,244 kg (1,79 Mol) reines Gemisch von α-Tocopherol-fettsäureestern als zähflüssiges gelbes Oel ergab (Gesamtausbeute 78%). Durch Analyse wurde folgende Zusammensetzung für dieses Gemisch festgestellt :

50,5 Gewichtsprozent α-Tocopherol-linolsäureester
29,5 Gewichtsprozent α-Tocopherol-ölsäureester
20 Gewichtsprozent Gemisch von α-Tocopherol-linolensäureester,
-palmitinsäureester, -myristinsäureester und -stearinsäureester.

Es wird dieses gereinigte Gemisch von α-Tocopherol-fettsäureestern in den Zusammensetzungen der nachfolgenden Beispiele verwendet.

Beispiel 2

## Lippenstift

| Bestandteile | | Gewichtsprozent |
|---|---|---|
| Gereinigtes Gemisch von α-Tocopherol-fettsäureestern des Beispiels 1 | | 3.0% |
| Candelillawachs | | 11.5% |
| Bienenwachs | | 9.0% |
| Cetylalkohol | | 5.0% |
| Petrolatum | | 15.0% |
| Butylstearat | | 5.0% |
| D & C Red No. 21 | Von der FDA für | 1.0% |
| D & C Red No. 19, "Barium Lake" | die Färbung von | 4.5% |
| D & C Red No. 11, "Calcium Lake" | Arzneimitteln | 2.5% |
| | und Kosmetika | |
| | zugelassene | |
| | rote Farbstoffe | |
| Rosenöl | | 0.5% |
| Rhizinusöl | | ad 100.0% |

Beispiel 3

## <u>Feuchthaltende Lotion</u>

| <u>Bestandteile</u> | | <u>Gewichtsprozent</u> |
|---|---|---|
| Gereinigtes Gemisch von α-Tocopherol-fettsäureestern des Beispiels 1 | | 5,0 % |
| Stearinsäure | | 4,0 % |
| Glycerylstearat und Polyäthylen-Glykol-(100)stearat | | 4,0 % |
| Isopropylpalmitat | | 1,0 % |
| Mineralöl | | 1,0 % |
| Propylparaben(4-Hydroxybenzoesäure-propylester) | | 0,1 % |
| Methylparaben(4-Hydroxybenzoesäure-methylester) | | 0,15% |
| Propylenglykol | | 3,0 % |
| Wasser | ad | 100,0 % |

Beispiel 4

## <u>Haarfestiger</u>

| <u>Bestandteile</u> | | <u>Gewichtsprozent</u> |
|---|---|---|
| Gereinigtes Gemisch von α-Tocopherol-fettsäureestern des Beispiels 1 | | 1 % |
| Stearalkoniumchlorid (Stearyl-dimethyl-benzylammoniumchlorid) | | 5 % |
| Hydroxypropyl-methylcellulose | | 1,5% |
| Cetylalkohol | | 1,5% |
| Wasser | ad | 100,0% |

Beispiel 5

## Festigendes Shampoo

| Bestandteile | Gewichtsprozent |
|---|---|
| Gereinigtes Gemisch von α-Tocopherol-fettsäureestern des Beispiels 1 | 0,75% |
| Ammoniumlaurylsulfat | 25,0 % |
| Cocoamidopropyl-Betain | 5,0 % |
| Lauramide DEA (Gemisch von Aethanolamiden der Laurinsäure) | 2,0 % |
| Propylparaben | 0,05% |
| Methylparaben | 0,20% |
| Wasser | ad 100,0 % |

Beispiel 6

## Lippenbalsam

| Bestandteile | Gewichtsprozent |
|---|---|
| Gereinigtes Gemisch von α-Tocopherol-fettsäureestern des Beispiels 1 | 3,0 % |
| Lanolinöl | 8,2 % |
| Bienenwachs | 30,0 % |
| Polyglycerol-4-oleat (Ester von Oelsäure und einem Glycerinpolymer) | 2,0 % |
| Paraffin | 15,0 % |
| BHT [2,6-Di(tert.Butyl)-4-methylphenol] | 0,06% |
| Fruktose | 2,0 % |
| Polysorbat-20 (Gemisch von Sorbitol-laurinsäureestern und Sorbitolanhydriden) | 2,0 % |
| Wasser | 10,0 % |
| Panthenol | 0,1 % |
| Glycerin | 2,0 % |
| Erdbeer-Geschmacksstoff | 0,1 % |
| Mineralöl | ad 100,0 % |

7

Beispiel 7

## Fetthaltende Crème

| Bestandteile | Gewichtsprozent |
|---|---|
| Gereinigtes Gemisch von α-Tocopherol-fettsäureestern des Beispiels 1 | 4,0 % |
| Stearinsäure | 2,2 % |
| Polyäthylenglykoläther von Oleylalkohol | 1,0 % |
| Glyceryl-Monostearat | 3,5 % |
| Isopropylpalmitat | 5,0 % |
| Polyäthylenglykoläther von Lanolinalkohol | 1,0 % |
| Bienenwachs | 1,5 % |
| Propylparaben | 0,1 % |
| Triäthanolamin | 1,0 % |
| Propylenglykol | 3,0 % |
| Wasser | 50,0 % |
| Methylparaben | 0,1 % |
| Mineralöl | ad 100,0 % |

Beispiel 8

## Körper-/Gesichtsöl

| Bestandteile | Gewichtsprozent |
|---|---|
| Gereinigtes Gemisch von α-Tocopherol-fettsäureestern des Beispiels 1 | 70% |
| Isopropylpalmitat | 30% |

## Ansprüche

1. Topisch applizierbares kosmetisches Mittel, das eine wirksame Menge eines feuchthaltenden Stoffes sowie kosmetisch verträgliche Trägerstoffe enthält, dadurch gekennzeichnet, dass der feuchthaltende Stoff aus einem Gemisch von α-Tocopherol-linolsäureester und einem oder mehreren anderen α-Tocopherol-fettsäuree-stern besteht, wobei diese Ester von gesättigten und/oder 1-3 Kohlenstoff-Kohlenstoff-Doppelbindungen auf-weisenden ungesättigten $C_{14-22}$-Fettsäuren abgeleitet sind.

2. Kosmetisches Mittel nach Anspruch 1, dadurch gekennzeichnet, dass der feuchthaltende Stoff den Linol-säureester und den Oelsäureester von α-Tocopherol enthält.

3. Kosmetisches Mittel nach Anspruch 2, dadurch gekennzeichnet, dass 30 bis 70 Gewichtsprozent des feuchthaltenden Stoffes aus α-Tocopherol-linolsäureester und 15 bis 40 Gewichtsprozent aus α-Tocopherol-ölsäureester bestehen.

EP 0 231 777 B1

4. Kosmetisches Mittel nach Anspruch 2, dadurch gekennzeichnet, dass der feuchthaltende Stoff neben α-Tocopherol-linolsäureester und α-Tocopherol-ölsäureester auch noch den Stearinsäure-, Palmitinsäure-, Linolensäure- und Myristinsäureester von α-Tocopherol enthält.

5. Kosmetisches Mittel nach Anspruch 4, dadurch gekennzeichnet, dass der feuchthaltende Stoff 30 bis 70 Gewichtsprozent α-Tocopherol-linolsäureester, 15 bis 40 Gewichtsprozent α-Tocopherol-ölsäureester, 2 bis 30 Gewichtsprozent α-Tocopherol-linolensäureester, 0,5 bis 20 Gewichtsprozent α-Tocopherol-palmitinsäureester, 0,1 bis 10 Gewichtsprozent α-Tocopherol-myristinsäureester und 0,1 bis 10 Gewichtsprozent α-Tocopherol-stearinsäureester enthält.

6. Kosmetisches Mittel nach einem der Ansprüche 1 bis 5, dadurch gekennzeichnet, dass neben dem feuchthaltenden Stoff einer oder mehrere weitere kosmetische Wirkstoffe, nämlich oberflächenaktive Mittel, Weichmacher, Farbstoffe, Haarfestiger, Bakterizide, Astringentien und Reinigungsmittel, vorhanden sind.

7. Kosmetisches Mittel nach Anspruch 6, dadurch gekennzeichnet, dass die Menge feuchthaltenden Stoffes von 0,1 bis 15 Gewichtsprozent der Gesamtmenge des Mittels beträgt.

8. Kosmetisches Mittel nach Anspruch 7, dadurch gekennzeichnet, dass die Menge feuchthaltenden Stoffes 1 bis 5 Gewichtsprozent der Gesamtmenge des Mittels beträgt.

9. Kosmetisches Mittel nach einem der Ansprüche 1 bis 5, dadurch gekennzeichnet, dass in diesem feuchthaltenden topisch applizierbaren kosmetischen Mittel die Menge des feuchthaltenden Stoffes 30 Gewichtsprozent bis 75 Gewichtsprozent der Gesamtmenge beträgt und in dem der kosmetisch verträgliche Trägerstoff ein Lösungsmittel ist, in dem der feuchthaltende Stoff gelöst oder emulgiert ist.

10. Kosmetisches Mittel nach Anspruch 9, dadurch gekennzeichnet, dass das Lösungsmittel aus einem oder mehreren Mineralölen, Fettsäure-niederalkylestern und Alkoholen besteht.

11. Gemisch von α-Tocopherol-fettsäureestern, dadurch gekennzeichnet, dass diese von gesättigten und-/oder 1-3 Kohlenstoff-Kohlenstoff-Doppelbindungen aufweisenden ungesättigten $C_{14-22}$-Fettsäuren abgeleitet sind und dass 30 bis 70 Gewichtsprozent des Gemisches aus α-Tocopherol-linolsäureester und 15 bis 40 Gewichtsprozent aus α-Tocopherol-ölsäureester bestehen.

12. Gemisch von α-Tocopherol-fettsäureestern nach Anspruch 11, dadurch gekennzeichnet, dass es α-Tocopherol-linolsäureester, α-Tocopherol-ölsäureester als auch den Stearinsäure-, Palmitinsäure-, Linolensäure- und Myristinsäureester von α-Tocopherol enthält.

13. Gemisch von α-Tocopherol-fettsäureestern nach Anspruch 12, dadurch gekennzeichnet, dass es 2 bis 30 Gewichtsprozent α-Tocopherol-linolensäureester, 0,5 bis 20 Gewichtsprozent α-Tocopherol-palmitinsäureester, 0,1 bis 10 Gewichtsprozent α-Tocopherol-myristinsäureester und 0,1 bis 10 Gewichtsprozent α-Tocopherol-stearinsäureester enthält.

14. Verwendung eines Mittels gemäss einem der Ansprüche 1 bis 10 oder eines Gemisches von α-Tocopherol-fettsäureestern gemäss einem der Ansprüche 11 bis 13 zur Feuchthaltung der Haut.

## Claims

1. A cosmetic composition for topical application, which contains an effective amount of a moisturizer as well as a cosmetically acceptable carrier material, characterized in that the moisturizer consists of a mixture of α-tocopherol linoleic acid ester and one or more other α-tocopherol fatty acid esters, whereby these esters are derived from saturated $C_{14-22}$-fatty acids and/or unsaturated $C_{14-22}$-fatty acids having 1-3 carbon-carbon double bonds.

2. A cosmetic composition according to claim 1, characterized in that the moisturizer contains the linoleic acid ester and the oleic acid ester of α-tocopherol.

3. A cosmetic composition according to claim 2, characterized in that 30 to 70 percent by weight of the moisturizer consists of α-tocopherol linoleic acid and 15 to 40 percent by weight of α-tocopherol oleic acid ester.

4. A cosmetic composition according to claim 2, characterized in that the moisturizer contains, apart from α-tocopherol linoleic acid ester and α-tocopherol oleic acid ester, also the stearic acid, palmitic acid, linolenic acid and myristic acid esters of α-tocopherol.

5. A cosmetic composition according to claim 4, characterized in that the moisturizer contains 30 to 70 percent by weight of α-tocopherol linoleic acid ester, 15 to 40 percent by weight of α-tocopherol oleic acid ester, 2 to 30 percent by weight of α-tocopherol linolenic acid ester, 0.5 to 20 percent by weight of α-tocopherol palmitic acid ester, 0.1 to 10 percent by weight of α-tocopherol myristic acid ester and 0.1 to 10 percent by weight of α-tocopherol stearic acid ester.

6. A cosmetic composition according to any one of claims 1 to 5, characterized in that, in addition to the moisturizer, one or more further cosmetically active ingredients, namely surfactants, emollients, colorants, hair conditioners, bacteriocides, astringents and detergents, are present.

9

7. A cosmetic composition according to claim 6, characterized in that the amount of moisturizer amounts to 0.1 to 15 percent by weight of the total weight of the composition.

8. A cosmetic composition according to claim 7, characterized in that the amount of moisturizer amounts to 1 to 5 percent by weight of the total weight of the composition.

9. A cosmetic composition according to any one of claims 1 to 5, characterized in that in this moisturizing cosmetic composition for topical application the amount of moisturizer amounts to 30 percent by weight to 75 percent by weight of the total weight and the cosmetically acceptable carrier material is a solvent in which the moisturizer is dissolved or emulsified.

10. A cosmetic composition according to claim 9, characterized in that the solvent is one or more mineral oils, fatty acid lower alkyl esters and alcohols.

11. A mixture of $\alpha$-tocopherol fatty acid esters, characterized in that these are derived from saturated $C_{14-22}$-fatty acids and/or unsaturated $C_{14-22}$-fatty acids having 1-3 carbon-carbon double bonds and in that 30 to 70 percent by weight of the mixture consists of $\alpha$-tocopherol linoleic acid ester and 15 to 40 percent by weight consists of $\alpha$-tocopherol oleic acid ester.

12. A mixture of $\alpha$-tocopherol fatty acid esters according to claim 11, characterized in that it contains $\alpha$-tocopherol linoleic acid ester, $\alpha$-tocopherol oleic acid ester as well as the stearic acid, palmitic acid, linolenic acid and myristic acid esters of $\alpha$-tocopherol.

13. A mixture of $\alpha$-tocopherol fatty acid esters according to claim 12, characterized in that it contains 2 to 30 percent by weight of $\alpha$-tocopherol linoleic acid ester, 0.5 to 20 percent by weight of $\alpha$-tocopherol palmitic acid ester, 0.1 to 10 percent by weight of $\alpha$-tocopherol myristic acid ester and 0.1 to 10 percent by weight of $\alpha$-tocopherol stearic acid ester.

14. The use of a composition in accordance with any one of claims 1 to 10 or of a mixture of $\alpha$-tocopherol fatty acid esters in accordance with any one of claims 11 to 13 for moisturizing the skin.

## Revendications

1. Composition cosmétique appliquable localement, contenant une quantité efficace d'un corps gardant l'humidité ainsi que des supports cosmétiquement acceptables, caractérisé en ce que le corps gardant l'humidité se compose d'un mélange d'ester d'acide linoléique-$\alpha$-tocophérol et d'un ou plusieurs autres esters d'acides gras d'$\alpha$-tocophérol, où ces esters sont dérivés d'acides gras en $C_{14}$ à $C_{22}$ saturés et/ou non saturés présentant de 1 à 3 doubles liaisons carbone-carbone.

2. Composition cosmétique selon la revendication 1, caractérisée en ce que le corps gardant l'humidité contient l'ester d'acide linoléique et l'ester d'acide oléique d'$\alpha$-tocophérol.

3. Composition cosmétique selon la revendication 2, caractérisée en ce que de 30 à 70% en poids du corps gardant l'humidité se composent d'ester d'acide linoléique-$\alpha$-tocophérol et de 15 à 40% en poids d'ester de l'acide oléique-$\alpha$-tocophérol.

4. Composition cosmétique selon la revendication 2, caractérisée en ce que le corps gardant l'humidité contient outre l'ester d'acide linoléique-$\alpha$-tocophérol et l'ester d'acide oléique-$\alpha$-tocophérol, les esters d'acides stéarique, palmitique, linolénique et myristique d'$\alpha$-tocophérol.

5. Composition cosmétique selon la revendication 4, caractérisée en ce que le corps gardant l'humidité contient de 30 à 70% en poids d'ester d'acide linoléique-$\alpha$-tocophérol de 15 à 40% en poids d'ester de l'acide oléique-$\alpha$-tocophérol de 2 à 30% en poids d'ester de l'acide linolénique-$\alpha$-tocophérol, de 0,5 à 20% en poids d'ester de l'acide palmitique-$\alpha$-tocophérol, de 0,1 à 10% en poids d'ester de l'acide myristique-$\alpha$-tocophénol et de 0,1 à 10% en poids d'ester de l'acide stéarique-$\alpha$-tocophérol.

6. Composition cosmétique selon l'une des revendications 1 à 5, caractérisée en ce qu'on y trouve, outre le corps gardant l'humidité, une ou plusieurs substances actives cosmétiques, à savoir des agents tensio-actifs, des plastifiants, des colorants, des fixateurs pour cheveux, des bactéricides, des agents astringents et nettoyants.

7. Composition cosmétique selon la revendication 6, caractérisée en ce que la quantité de corps gardant l'humidité s'élève de 0,1 à 15% en poids de la quantité totale de la composition.

8. Composition cosmétique selon la revendication 7, caractérisée en ce que la quantité de corps gardant l'humidité s'élève de 1 à 5% en poids de la quantité totale de la composition.

9. Composition cosmétique selon l'une des revendications 1 à 5, caractérisée en ce que dans ces compositions cosmétiques gardant l'humidité applicables localement la quantité de corps gardant l'humidité s'élève de 30% en poids à 75% en poids de la quantité totale et en ce que le support cosmétiquement acceptable est un solvant dans lequel le corps gardant l'humidité est dissous ou émulsifié.

10. Composition cosmétique selon la revendication 9, caractérisée en ce que le solvant se compose d'une ou plusieurs huiles minérales, alcoyle-inférieur-esters d'acides gras et alcools.

11. Mélange d'esters d'acides gras d'α-tocophérol, caractérisé en ce qu'ils sont dérivés d'acides gras en $C_{14}$ à $C_{22}$ saturés et/ou non saturés présentant de 1 à 3 doubles liaisons carbone-carbone, et en ce qu'ils se composent de 30 à 70% en poids du mélange d'ester d'acide linoléique-α-tocophérolet de 15 à 40% en poids d'ester d'acide oléique-α-tocophérol.

12. Mélange d'esters d'acide gras d' α-tocophérol selon la revendication 11, caractérisé en ce qu'il contient l'ester de l'acide linoléique-α-tocophérol, l'ester de l'acide oléique-α-tocophérol ainsi que les esters d'acides stéarique, palmitique, linolénique et myristique d'α-tocophérol.

13. Mélange d'esters d'acides gras d'α-tocophérol selon la revendication 12, caractérisé en ce qu'il contient de 2 à 30% en poids d'ester de l'acide linolénique-α-tocophérol, de 0,5 à 20% en poids d'ester de l'acide palmitique-α-tocophérol, de 0,1 à 10% en poids d'ester de l'acide myristique-α-tocophérol et de 0,1 à 10% en poids d'ester de l'acide stéarique-α-tocophérol.

14. Application d'une composition selon l'une des revendications 1 à 10 ou d'un mélange d'esters d'acides gras d'α-tocophérol selon l'une des revendications 11 à 13 pour garder l'humidité de la peau.